Europäisches Patentamt

European Patent Office

Office européen des brevets

(11) Veröffentlichungsnummer: **0 301 554 B1**

# EUROPÄISCHE PATENTSCHRIFT

(45) Veröffentlichungstag der Patentschrift: **13.11.91**

(51) Int. Cl.5: **G01N 33/53**, G01N 33/52

(21) Anmeldenummer: **88112262.6**

(22) Anmeldetag: **28.07.88**

(54) **Verfahren zur Beseitigung von unspezifischen Trübungen.**

(30) Priorität: **31.07.87 DE 3725475**

(43) Veröffentlichungstag der Anmeldung:
**01.02.89 Patentblatt 89/05**

(45) Bekanntmachung des Hinweises auf die
Patenterteilung:
**13.11.91 Patentblatt 91/46**

(84) Benannte Vertragsstaaten:
**AT BE CH DE ES FR GB GR IT LI LU NL SE**

(56) Entgegenhaltungen:
**EP-A- 0 141 879**
**EP-A- 0 141 922**
**DE-A- 3 324 626**

(73) Patentinhaber: **BOEHRINGER MANNHEIM GMBH**
**Patentabteilung, Abt. E Sandhofer Strasse**
**112-132 Postfach 31 01 20**
**W-6800 Mannheim 31 Waldhof(DE)**

(72) Erfinder: **Zdunek, Dietmar, Dr.rer.nat.**
**Noestr. 23**
**W-8000 München 71(DE)**
Erfinder: **Weber, Friederike**
**Rheinstr. 33**
**W-8000 München 40(DE)**

(74) Vertreter: **Huber, Bernhard, Dipl.-Chem. et al**
**Möhlstrasse 22 Postfach 860 820**
**W-8000 München 86(DE)**

**Beschreibung**

Die Erfindung betrifft ein Verfahren zur Beseitigung von unspezifischen Trübungen bei der Durchführung von Bestimmungen nach dem Prinzip des Immunoassays.

Die Entwicklung von Bestimmungen nach dem Prinzip des Immunoassays hat zu einer entscheidenden Verbesserung der Empfindlichkeit und Selektivität bei der Bestimmung von spezifisch bindefähigen Substanzen wie Proteinen, Haptenen und Antikörpern geführt und daher insbesondere in den klinisch chemischen Laboratorien große Bedeutung erlangt. Die Weiterentwicklung dieser Methoden ausgehend vom Radioimmunassay führte neben der Verbreiterung der zur Verfügung stehenden Markierungsmethoden auch zur Entwicklung verschiedener Analysentechniken. Weitverbreitet sind heute Immunoassays, die Enzyme oder fluoreszierende Verbindungen als Markierung verwenden. Die Bestimmung der Markierung und damit der zu untersuchenden Substanz beruht dabei auf einer photometrisch zu erfassenden Absorptionsänderung oder Emission von Licht. Grundvoraussetzung dieser Verfahren ist es, daß sie ein Meßsignal liefern, das der Menge an zu bestimmender Substanz direkt proportional ist, wobei gleichzeitig eine Störung durch Komponenten, die in der zu untersuchenden Lösung, wie z. B. dem Serum, enthalten sind, mit Sicherheit vermieden werden muß und daß sie außerdem eine sehr hohe Empfindlichkeit aufweisen, so daß auch noch im Pico-Molbereich ausreichend genaue Ergebnisse erzielt werden können. Es wurde durch diese neuen Bestimmungsverfahren möglich, Substanzen im Serum nachzuweisen, deren Nachweis mit den früher bekannten analytischen Verfahren überhaupt nicht möglich war.

Beim Nachweis dieser in sehr geringen Mengen vorliegenden Substanzen, wie beispielsweise TSH oder AFP, ist es natürlich besonders wichtig, jede Störung, die zu einer Verfälschung des Ergebnisses führt, auszuschalten. Bei der Bestimmung klinischer Parameter in Serumproben bzw. in Standards, die auf Serumbasis hergestellt sind, treten nun häufig Störungen auf durch unspezifische Agglutination. Diese unspezifische Agglutination führt zu mehr oder weniger geringen Trübungen, die die photometrische Auswertung stören. Diese unspezifischen Agglutinationen konnten bisher weder durch Einsatz chaotroper Reagenzien, noch durch Zusatz von Detergentien vollständig eliminiert werden.

Es war daher Aufgabe der vorliegenden Erfindung, ein Verfahren zu schaffen, um unspezifische Agglutinationen in Serumproben zu unterdrücken bzw. aufzulösen.

Diese Aufgabe wird gelöst durch ein Verfahren zur Beseitigung von unspezifischen Trübungen bei der Durchführung von Bestimmungen nach dem Prinzip des Immunoassays, das dadurch gekennzeichnet ist, daß man der Probe ein anorganisches Borsäurederivat in Kombination mit einem Puffersystem zusetzt.

Überraschenderweise wird durch die Anwendung einer Kombination der beiden erfindungsgemäßen Additive die unspezifische Agglutination vollständig unterdrückt, so daß bei der photometrischen Bestimmung von Substanzen, die in niedrigsten Konzentrationen im Serum vorliegen, keine Störungen mehr auftreten.

Bei der Durchführung von Bestimmungen nach dem Prinzip des Immunoassays, die an sich bekannt sind, wird erfindungsgemäß der Probenlösung eine Kombination aus einem anorganischen Borsäurederivat und einem Puffersystem zugesetzt. Damit können unspezifische Trübungen beseitigt werden. Der Zusatz der erfindungsgemäßen Substanzen erfolgt vor der photometrischen Messung und kann zusammen mit den für die Durchführung des Immunoassays verwendeten Reagenzien erfolgen oder aber auch vorher dem Serum zugesetzt werden oder nach Zugabe der Reagenzien. Wichtig ist nur, daß die Beseitigung der Trübung vor Durchführung der photometrischen Bestimmung erfolgt.

Eine wesentliche Komponente ist ein anorganisches Borsäurederivat. Hier gibt es eine Vielzahl von Verbindungen, die erfindungsgemäß geeignet sind. Sie sind handelsüblich, stabil und im Reaktionssystem löslich. Besonders bevorzugt werden Borat, Metaborat, Tetraborat und Perborat in Form ihrer Alkalisalze, bevorzugt als Natriumsalze verwendet.

Die Borate, Metaborate und Tetraborate sind für alle Immunoassays geeignet. Perborat ist als Borverbindung sehr gut wirksam, es sollte jedoch nur bei Immunoassays eingesetzt werden, in denen oxidationsstabile Antikörper verwendet werden. Bei Immunoassays, bei denen oxidationsempfindliche Antikörper eingesetzt werden müssen, wie beispielsweise der TSH-Antikörper, wird durch den Einsatz des Perborats die Antikörperreaktion beeinflußt. In diesem Falle sollte daher eine andere Borverbindung verwendet werden.

Das anorganische Borsäurederivat wird bevorzugt in einer Konzentration von 100 bis 150 mmol/l verwendet. Bei einer Verwendung von weniger als 100 mmol/l Borat ist unter Umständen die Beseitigung der Trübung nicht mehr optimal. Eine Menge von mehr als 150 mmol/l Borat bringt keine weitere Verbesserung mehr, so daß es unzweckmäßig ist, höhere Konzentrationen zu verwenden.

Als zweite Komponente wird der Probenlösung ein von Boratpuffer verschiedenes Puffersystem zugesetzt. Geeignet sind hier die für immunologische Verfahren bekannten Puffersysteme. Bevorzugt werden Puffer des GOOD-Puffersystems, wie Tris- oder Hepespuffer und Phosphatpuffer verwendet. Besonders bevorzugt wird erfindungsgemäß Trispuffer eingesetzt.

Das Puffersystem wird in einer Konzentration von 70 bis 100 mmol/l eingesetzt. Geringere Konzentrationen führen nicht zu einem optimalen Ergebnis, wogegen mehr als 100 mmol/l Puffer keine weitere Verbesserung mehr bewirken können.

Das erfindungsgemäße Verfahren eignet sich für alle Arten von Immunoassays, bei denen eine photometrische Bestimmung erfolgt, wie ELISA oder LPIA. Insbesondere ist das erfindungsgemäße Verfahren geeignet für den Nachweis von Substanzen, die in sehr geringen Konzentrationen vorliegen.

Die Erfindung wird noch durch die folgenden Beispiele erläutert.

**Beispiel 1**

Es wurden Immunoassays zur Bestimmung von AFP durchgeführt unter Verwendung von verschiedenen Puffersubstanzen und verschiedenen Borsäurederivaten. Die folgenden Reagenzien wurden verwendet:

Reagenz 1:

100 mmol/l Puffer, pH 7,5,

120 mmol/l Borsäurederivaten,

2 Gew.-% Polyethylenglykol mit einem durchschnittlichen Molekulargewicht von ca. 6000 (PEG 6000);

0,5 Gew.-% Pluronic F68® (Tensid auf Basis von Alkylenoxid, Poloxamer);

1 Gew.-% Rinderserumalbumin.

Als Reagenz 2 wurde eine Latexpartikeldispersion verwendet, die wie folgt hergestellt wurde:

Die Herstellung erfolgte nach der Carbodiimidmethode, wie sie in J. EX. med. (1981) 154-1, 1539-1553; US-P 3 857 931 sowie J. Immunol. Meth. (1978), 22, 165-174 beschrieben ist. Dazu wurden Polystyrolpartikel, die einen Durchmesser von 70 bis 300 nm hatten und aktivierte Carboxylgruppen aufwiesen, mit polyklonalen Antikörpern gegen AFP mit einer Beladungsdichte von 3 bis 400 µg Antikörper pro mg Latex je nach Partikelgröße umgesetzt. Anschließend wurde dreimal in 200 mmol/l Glycinpuffer, pH 7,5 + 1 % Rinderserumalbumin gewaschen und zentrifugiert. 80 µ1 einer Dispersion von 0,35 Gew.-% dieser Latexpartikel in 200 mmol/l Glycinpuffer, pH 7,5 wurden jeweils eingesetzt.

Als Probe wurde Humanserumstandard, der analytfrei war, verwendet.

Es wurden nun jeweils 860 µ1 Reagenz 1 und 100 µ1 Probe bei 37°C zusammengegeben und der Leerwert bei 623 nm bestimmt. Anschließend wurden 80 µ1 Reagenz 2 zugegeben und nach 5 Minuten die Turbidität bei 623 nm gemessen. Das Ergebnis ist der Tabelle I zu entnehmen. Es zeigt sich, daß die unspezifische Agglutination bei Zusatz der erfindungsgemäßen Kombination eines Puffers mit einer Borverbindung bis zu 100 % inhibiert wird, während der Zusatz von Puffer oder einer Borverbindung alleine die Trübung nicht beseitigen kann.

**Beispiel 2**

Es wurden mehrere Immunoassays zur Bestimmung von TSH durchgeführt, wobei jeweils als Puffer Trispuffer verwendet wurde und das Borsäurederivat variiert wurde. Es wurde im wesentlichen wie im Beispiel 1 verfahren, wobei Reagenz 1 jedoch die folgende Zusammensetzung hatte:

70 mmol/l Puffer, pH 7,0,

120 mmol/l Borsäurederivat,

2 Gew.-% PEG 6000,

0,5 Gew.-% Pluronic F68®,

1 Gew.-% Rinderserumalbumin,

18 mmol/l EDTA,

10 mmol/l Natriumthiocyanat.

Als Antikörper wurden polyklonale Antikörper gegen TSH verwendet. Die Ergebnisse sind in Tabelle 2 angegeben. Auch hier zeigt sich, daß eine Kombination von Trispuffer mit Borat zu einer praktisch vollständigen Unterdrückung der unspezifischen Agglutination führt.

## T a b e l l e   1

Beispiel: TSH-Bestimmung

Konzentration des Puffer: 70 mmol/l im Test

Konzentration der Bor-Verbindung: 120 mmol/l im Test

| Puffer | Unspezifische Agglutination mE/5min | | % Inhibition unspez. Aggl. |
|---|---|---|---|
| | Puffer | Puffer+Metaborat | |
| TRIS Metaborat | 142 106 | 76 -- | 47 -- |
| | Puffer | Puffer+Borsäure | |
| TRIS Borsäure | 120 230 | 10 -- | 92 -- |
| | Puffer | Puffer+Perborat | |
| TRIS Perborat | 142 92 | 0 -- | 100 -- |

T a b e l l e    2

Beispiel: TSH-Bestimmung

Konzentration des Puffer:            70 mmol/l im Test

Konzentration der Bor-Verbindung: 120 mmol/l im Test

| Puffer | Unspezifische Agglutination mE/5min | | % Inhibition |
|---|---|---|---|
| | Puffer | Puffer+Metaborat | |
| Tris Metaborat | 142 106 | 76 -- | 47 -- |
| | Puffer | Puffer+Borsäure | |
| Tris Borsäure | 120 230 | 10 -- | 92 -- |
| | Puffer | Puffer+Perborat | |
| Tris Perborat | 142 92 | 0 - | 100 -- |

**Patentansprüche**

1. Verfahren zur Beseitigung von unspezifischen Trübungen bei der Durchführung von Bestimmungen nach dem Prinzip des Immunoassays,
**dadurch gekennzeichnet,**
daß man der Probenlösung ein anorganisches Borsäurederivat in Kombination mit einem Puffersystem zusetzt.

2. Verfahren nach Anspruch 1, **dadurch gekennzeichnet,** daß man als Borsäurederivat ein Borat, Metaborat, Tetraborat oder Perborat verwendet.

3. Verfahren nach einem der vorhergehenden Ansprüche, **dadurch gekennzeichnet,** daß man die Borverbindung in einer Menge von 100 bis 150 mmol/l verwendet.

4. Verfahren nach einem der vorhergehenden Ansprüche, **dadurch gekennzeichnet,** daß man als Puffersystem einen GOOD-Puffer oder einen Phosphatpuffer verwendet.

**5.** Verfahren nach Anspruch 4, **dadurch gekennzeichnet,** daß man als GOOD-Puffer Tris- oder HEPES-Puffer verwendet.

**6.** Verfahren nach einem der vorhergehenden Ansprüche, **dadurch gekennzeichnet,** daß man das Puffersystem in einer Menge von 70 bis 100 mmol/l verwendet.

## Claims

**1.** Process for the removal of non-specific turbidities in the carrying out of determinations according to the principle of immunoassay, characterised in that one adds to the sample solution an inorganic boric acid derivative in combination with a buffer system.

**2.** Process according to claim 1, characterised in that one uses a borate, metaborate, tetraborate or perborate as boric acid derivative.

**3.** Process according to one of the preceding claims, characterised in that one uses the boron compound in an amount of 100 to 150 mmole/l.

**4.** Process according to one of the preceding claims, characterised in that one uses a GOOD buffer or a phosphate buffer as buffer system.

**5.** Process according to claim 4, characterised in that one uses tris or HEPES buffer as GOOD buffer.

**6.** Process according to one of the preceding claims, characterised in that one uses the buffer system in an amount of 70 to 100 mmole/l.

## Revendications

**1.** Procédé pour l'élimination d'opacités non spécifiques lors de l'accomplissement de déterminations selon le principe de l'immunodosage,
caractérisé en ce que l'on ajoute à la solution échantillon un dérivé minéral de l'acide borique en combinaison avec un système tampon.

**2.** Procédé selon la revendication 1, caractérisé en ce que l'on utilise comme dérivé de l'acide borique un borate, métaborate, tétraborate ou perborate.

**3.** Procédé selon l'une des revendications précédentes, caractérisé en ce que l'on utilise le composé du bore en une quantité de 100 à 150 mmol/l.

**4.** Procédé selon l'une des revendications précédentes, caractérisé en ce que l'on utilise comme système tampon un tampon GOOD ou un tampon phosphate.

**5.** Procédé selon la revendication 4, caractérisé en ce que l'on utilise comme tampon GOOD un tampon tris ou HEPES.

**6.** Procédé selon l'une des revendications précédentes, caractérisé en ce que l'on utilise le système tampon en une quantité de 70 à 100 mmol/l.